# EUROPEAN PATENT SPECIFICATION

(11) **EP 1 054 678 B1**
(45) Date of publication and mention of the grant of the patent: **28.05.2003**
(21) Application number: 99906801.8
(22) Date of filing: 10.02.1999
(51) Int. Cl.: A61K 31/585, A61K 35/60, A61K 31/20, A61P 1/00

(54) **COMBINATION OF STEROID AND POLYUNSATURATED FATTY ACIDS FOR TREATMENT OF INFLAMMATORY CONDITIONS**
UNGESÄTTIGTE FETTSÄURE UND STEROIDE ENTHALTENDE KOMBINATIONSPRÄPARATE ZUR BEHANDLUNG VON ENTZÜNDUNGEN
COMPOSITION COMBINANT DES ACIDES GRAS POLYINSATURES ET DES STEROIDES POUR LE DE TRAITEMENT D'ETATS INFLAMMATOIRES

(30) Priority: 11.02.1998 US 22008; 08.02.1999 US 245912
(43) Date of publication of application: 29.11.2000
(73) Proprietor: RTP Pharma Corporation, Durham, North Carolina 27713-2280 (US)
(72) Inventor: GUIVARC'H, Pol-Henri, Montreal, Quebec H3L 3C1 (CA); ROBINSON, Gary, Montreal, Quebec H2L 2P5 (CA)
(74) Representative: Bassil, Nicholas Charles
(86) International application number: US9902671
(87) International publication number: WO99040906

(56) References cited:
- WO-A-96/34846
- US-A- 5 863 910
- SALOMON P ET AL: "Treatment of ulcerative colitis with fish oil n-3 omega fatty acid: an open trial" J CLIN GASTROENTEROL, vol. 12, no. 2, 1990, pages 157-151, XP002111586
- HAWTHORNE A. B. ET AL: "Treatment of ulcerative colitis with fish oil supplementation: a prospective 12 month randomised controlled trial" GUT, vol. 33, 1992, XP002111587
- LOESCHKE K. ET AL: "n-3 fatty acids only delay early relapse of ulcerative colitis in remission" DIGESTIVE DISEASES AND SCIENCES, vol. 41, no. 10, October 1996 (1996-10), pages 2087-2094, XP002111588
- BELLUZZI A.: "Effect of an enteric-coated fish oil preparation on relapses in Crohn's disease" NEW ENGLAND JOURNAL OF MEDICINE, vol. 334, no. 24, 13 June 1996 (1996-06-13), pages 1557-1560, XP002111589
- THOMSON ABR ET AL: "Budesonide in the management of patients with Crohn's disease" CAN J. GASTROENTEROL, vol. 11, no. 3, March 1997 (1997-03), XP002111590

## Description

This invention provides compositions for preventing, mitigating, or treating inflammation of the gastrointestinal tract and systemic or local symptoms of inflammation, which contain an anti-inflammatory drug, in combination with a source of polyunsaturated fatty acids, such as omega-3, omega-6, and omega-9 polyunsaturated fatty acids, and also a source of a pharmacologically active antioxidant such as tocopherols (e.g., alpha- and/or gamma-tocopherol). Also described are uses, for the manufacture of a medicament for preventing, mitigating, or treating such inflammation, of oral, local or otherwise administrable compositions of this invention.

### Background of the Invention

The present invention relates to a composition treating inflammatory conditions of "the gastrointestinal tract" (GIT); for those conditions that may be distant from, but sequelae of, GIT; and for systemic or localized (i.e., non-intestinal) inflammations not related to GIT inflammation.

Inflammation (defined below) can be chronic or acute, or can alternate between the two states. Inflammation of the GIT may be due to etiologies as diverse as infection, reaction to drugs or other foreign (irritating) substances, or to diseases such as Inflammatory Bowel Diseases (Crohn's disease; ulcerative colitis). The primary focus of this invention is IBD, but is intended only as an example and not as a restriction or limitation to that disease, site or form of inflammation.

As an example of a disease addressed by the present invention. IBD is characterized by periods of varying disease activity, i.e.. quiescent, intermediate, and acute (active) phases. Depending on the phase symptoms can range from none, to mild and somewhat tolerable, to severe and requiring hospitalization for treatment. The etiology of the disease is unknown, but the principal pathophysiology seems to be the result of a "hyper-inflammatory" condition within the GIT. During the intermediate and acute phases, extra-intestinal (systemic) involvement may occur, e.g., ophthalmic, arthritic. The systemic conditions themselves are inflammatory in pathophysiology, and present discrete management approaches.

Treatment of chronic and acute aspects of IBD include, but is not limited to, drug therapy. During the quiescent phases, i.e., during remission, some patients require no medical treatment, although dietary management is often instituted as adjunctive therapy (see below). In the intermediate stages, patients experience mild symptoms which can be rendered tolerable with low (maintenance) doses of various medications. Drugs employed include both steroidal and non-steroidal anti-inflammatory drugs (e.g., steroids, such as prednisone and prednisolone), as well as derivatives of 5-aminosalicylic acid e.g., Mesalamine, Sulphasalazine, Olsalazine, and Balsalazide), and other drugs known collectively as "immunosuppresives" (e.g., Cyclosporine, Azathioprine, and 6-Mercaptopurine). Finally, adjunctive diet therapy is usually instituted.

For active stages of Inflammatory Bowel Disease, for example in Crohn's disease, intensive drug therapy is indicated, sometimes in a hospital setting. Steroids and immunosuppressive drugs are employed, in higher doses than in chronic phases. A new steroid, budesonide (Astra) has recently been introduced. Rutgeerts (1994) have shown budesonide to be nearly as effective, and with fewer side-effects compared to prednisone, in the acute phase of Crohn's Disease (CD). Budesonide is especially useful because it is delivered "topically", i.e., to the luminal side of the GIT, which in CD is the site of inflammation; this reduces undesirably high concentrations of the drug in the systemic circulation, which can lead to adverse manifestations of the drug. Further, the provision of budesonide, or other topically active drugs, in a sustained release formulation would be a preferred treatment.

Adjunctive therapy usually includes dietary modifications, which are variable in type and extent. For patients in remission, medical treatment is not employed. However, some efforts have been made to delay the onset of relapse to the active states. Such efforts often employ dietary treatment. For example, patients may limit themselves to diets low in residue (fiber) and "bland" diets. A recent study demonstrated that patients who consumed a source of omega-3 polyunsaturated fatty acids daily for one year suffered fewer relapses than those taking placebo oil. For those with intermediate symptoms, low-dose medications may be combined with dietary restrictions similar to those noted above.

Some patients have such severe acute phases that total bowel rest is employed, wherein nutrition is provided in the form of total parenteral nutrition (TPN). Others may do well - perhaps as well as those on drugs - with special diets known as "medical foods". Medical foods are usually liquid, are defined formulas, are intended for dietary management of specific diseases, and are administered under the supervision or a physician. Since the chronic phases of the disease often lead to partial anorexia, and may include malabsorption, patients not uncommonly manifest degrees of malnutrition. Such malnutrition can include deficiencies in essential fatty acids (EFA). and dietary therapy often includes good sources of EFA. Once stabilized, patients may be placed on restricted diets (low in residue; "bland"). Only when remission is complete are "normal" diets introduced.

The "therapeutic" (i.e., induction of remission) properties of some medical foods has led to speculation as to the identity and the mechanism of action of the responsible dietary components. Many food components of such diets have received attention in this regard, especially the lipid components known as "omega-3 polyunsaturated fatty acids" ("omega-3") and derivatives such as triglycerides and esters, which themselves have anti-inflammatory metabolic properties. In fact, omega-3 have been shown to have anti-inflammatory effects in clinical trials of asthma, rheumatoid arthritis, and cancer. A recent clinical trial showed that omega-3 slowed the rate of relapse in patients with "quiet" Crohn's disease (Belluzzi, et al. 1996). This is consistent with an anti-inflammatory effect of omega-3. Other clinical studies have shown benefit of omega-3 supplementation in intestinal diseases (Stenson et al., 1992. Mate et al., 1991; and other references in Belluzzi et al., 1996). Further still, other studies using lipid sources rich in omega-6 and omega-9 polyunsaturated fatty acids have also suggested that patients suffering from inflammatory conditions can gain symptomatic relief from appropriate dietary supplementation. Whilst the primary focus of this invention is the combination of anti-inflammatory drugs with refined edible oils enriched with omega-3 polyunsaturated fatty acids, other sources of enriched polyunsaturated fatty acids, such as omega-6 and omega-9 polyunsaturated fatty acids, can substitute in formulations for omega-3. The use of omega-3 throughout this invention is intended only as an example and not as a restriction or limitation to the specifications of drug and oil formulations.

The addition of antioxidants to the invention can provide both clinical and formulation benefits. As an example, but not a limitation to our invention, the nutrient known categorically as "vitamin E" consists of a mixture of isomers of tocopherol, of which the alpha and gamma forms are the most common in blood and in most foods. Tocopherols are potent antioxidants *in vitro,* and are usually added to preparations of polyunsaturated fatty acids to minimize oxidation. Tocopherols also exert beneficial effects *in vivo*, in animals and man, due possibly to their antioxidant properties. We are not aware that tocopherols have been studied with regard to GIT inflammation. their beneficial clinical effects give a second reason for adding them, beyond the issue of formulation stability. It was recently shown that the alpha and gamma isomers of the tocopherols differ in their specificity of antioxidant capacity, that is, a combination of the two forms would theoretically give better overall protection against excessive oxidation.

A combination of drug and the nutrients, omega-3, and of tocopherols, may improve the efficacy of treatment in IBD and other inflammatory conditions, in greater than additive fashion, which is the subject of the present invention.

### Summary of the Invention

The present invention provides compositions for treating the clinical manifestations of chronic or acute inflammation of the GIT, and/or of non-GIT sites in the body. The compositions include a source of polyunsaturated fatty acids. such as omega-3, and/or omega-6, and/or omega-9 fatty acids, including their triglycerides and esters; a drug used to treat the medical condition and a source of pharmacologically active antioxidant (preferably a combination of the alpha- and gamma-isomers of tocopherol). Optionally also included are pharmaceutically acceptable carriers, including other essential and non-essential oils, diluents, viscosity-modifiers, stabilizers, erodable or swallowable matrices, and penetration enhancers.

### Detailed Description of the Invention

The present invention provides an oral, enteral, or topical formulation comprising a source or form of polyunsaturated fatty acids, e.g. omega-3, and a source or form of pharmacologically active antioxidant such as tocopherols, and an anti-inflammatory steroid drug such as budesonide, for the treatment of inflammatory conditions of the GIT and/or of non-GIT sites in the body. The formulation combines the anti-inflammatory agent, e.g. budesonide, with an appropriate highly purified polyunsaturated fatty acid source, e.g., omega-3, or combination of highly purified or refined oils, that can provide for a formulation containing fully solubilized drug or varying degrees of suspended drug. Changes in the amount of fatty acid enrichment, source of oil, type(s) of oil used (e.g., free fatty acid, ethyl ester, or triglyceride), and/or addition of pharmaceutically acceptable excipients, such as carriers, including other essential and non-essential oils, diluents, viscosity-modifiers, stabilizers, erodable or swallowable matrices, and penetration enhancers can be used to modify the solubility of the drug in the formulation. In addition, the same methods can be used to enhance or decrease the uptake of drug and/or oil components of the formulations, as well as modify various physical characteristics of the formulation such as viscosity, spread, and residence time. This invention specifically teaches that the delivery of drug, for example to the intestinal tract, can be controlled by the addition of appropriate excipients, as detailed above, or primarily by modifying the characteristics of the formulation using various types, or combinations of various types, of highly purified polyunsaturated fatty acids. The modification would be tailored to suit the chosen disease target, i.e., the type, location, and intensity of the inflammatory reaction present, as detailed in the examples. Preferably, but not exclusively, formulations are preferred that use highly refined oils that have inherent anti-inflammatory properties, e.g. the omega-3, omega-6, and omega-9 families of polyunsaturated fatty acids. The formulation thus effectively prevents or reduces acute flare-ups of certain such diseases; reduces the dose of drug required for effectiveness; and reduces the side effects associated with the drugs. The formulation also reduces the incidence or severity of the chronic systemic effects of the disease. Finally, the formulation reduces the incidence or severity of side effects of the drug component of the therapy. The action of the omega-3 and/or omega-6 and/or omega-9 and antioxidant components, or the combination of all ingredients, may allow a lower initial dose, or final "tapered" dose. or maintenance dose of the drug component than would normally be prescribed and effective. This lessening of dosage of the drug. without compromising clinical efficacy, is part of the invention.

Surprisingly we have also found the presence of oils such as those enriched for omega-3 polyunsaturated fatty acids, quite apart from their potential therapeutic benefit, act as a carrier for the anti-inflammatory drug. In some instances the highly purified oils, e.g. omega-3, serve as a suspending medium for providing a slow drug release and in other cases they partially or fully solubilize the drug providing for faster drug release or drug uptake. The modification of the solubility of drug in the formulation is possible by addition of various agents, as detailed above, or the use of different types or combination of different types of highly purified oils.

If omega-3 oils are used, added benefits of the formulation include, firstly, the repletion of EFA, which have been shown to be lower than normal in some patients with inflammatory diseases of the intestine. Secondly, since the status of tocopherols (Vitamin E) is also often compromised under conditions of malnutrition or intestinal disease, an added benefit is the repletion of this vitamin with the formulation. Finally. tocopherols act as an antioxidant to protect polyunsaturated fatty acids.

The following terms used throughout this specification and in the appended claims arc defined as follows: "treating" refers to any or all of the following:
- prevention of the occurrence of a relapse, i.e., maintenance of remission
- induction of remission of acute phases of GIT inflammation, regardless of etiology
- either of the above in patients who are refractory to other medical treatments, and/or who are steroid-dependent or dependent on other medication
- prevention or reduction of the effects of the disease, whether expressed in the GIT or elsewhere, i.e., systemically or locally
- reduction of the required dose of drug whether in acute or non-acute phases
- reduction of the side effects of the drug component of therapy

"Inflammation" refers here to redness, heat, swelling and pain in a local area of the body, often with pain or disturbed function, in reaction to an infection, or to a physical or chemical injury. Further, and relevant to this invention, biochemical mediators and effectors of inflammation include "eicosanoids", which are produced by white cells from essential fatty acids, including omega-3. It is known that the eicosanoids produced from omega-3 are much less inflammatory, or anti-inflammatory, in their actions. These elcosanoids may also be "immuno-suppressive". Eicosanoid production from omega-3 polyunsaturated fatty acids accounts, in part, for their anti-inflammatory properties. However, other physiological and biochemical properties of omega-3 polyunsaturated fatty acids could contribute to their salutory effects in inflammation. The anti-inflammatory properties of the omega-6 and omega-9 polyunsaturated fatty acids are much less well documented, but offer similar potential benefit through somewhat different but nevertheless overlapping mechanisms.

"GIT inflammation" refers here to the condition of inflammation occurring or threatening to occur in any portion of the GII, from mouth to anus.

"Systemic or local inflammation" refers here to the condition of inflammation occurring or threatening to occur in any site of the body other than the GIT including the skin.

The drug or drugs present in the compositions and employed in the methods of this invention are anti-inflammatories as currently used in the treatment of inflammatory bowel diseases. Suitable anti-inflammatory drugs include corticosteroids such as prednisone, hydrocortisone, tixocortol, beclamethasone, budesonide.

"Omega-3" refers here to oils containing any or all polyunsaturated fatty acids of that chemical designation and their derivatives including triglyceridcs and esters. including but not limited to eicosapentaenoic acid (EPA) and docosahexaenoic acid (DHA). The fatty acids will be in the form of tri-, di-, or mono-glycerides, or in their "free" forms, such as ethyl esters. Sources include: animals (e.g., fish); plants (e.g., evening primrose. borage); other living sources, such as algae, bacteria, and yeast and their "bioengineered" derivative-forms; preparations that have been purified, modified or synthesized; and precursors of "omega-3". Included as well will be derivatives that may be water-soluble. ''Omega-6" and "Omega-9" refers here to oils containing any or all polyunsaturated fatty acids of that chemical designation and their derivatives as detailed for omega-3. The omega-3, omega-6, and omega-9 polyunsaturated fatty acids may typically be administered in amounts ranging from 1 to 6 grams per day. possibly up to 30 grams per day, but usually about 3 grams per day for most indications. However, since the exact amount of polyunsaturated fatty acids administered would be dependent upon the purity of the omega-3, omega-6, or omega-9 source, the site of drug administration, the relative amount of the anti-inflammatory drug used in combination with polyunsaturated fatty acids, and the indication being treated, the actual amount of polyunsaturated fatty acids used could be much lower than 1g per day.

"Antioxidants" refers here to any naturally-occurring or synthetic chemicals which are known or believed to exert antioxidant effects *in vitro* and/or *in vivo*. that is pharmacologically active. Examples include several vitamins, such as tocopherols. ascorbate, and beta-carotene.

"Tocopherol" refers here to any source, form, isomer, or derivative of tocopherols ("vitamin E"), especially mixtures of the alpha- and gamma-isomers.

Vitamin E, which is usually D-alpha-tocopherol is available as a dietary supplement in 100, 400 and 1,000 IU capsules. For purposes of the present invention a usual daily dosage is about 400IU. However, since the design of the invention is to be able to taper any one, or all of the components to facilitate intervention in the inflammatory process, the levels of antioxidant used may start from significantly lower than the daily dosage amount or may be absent. Furthermore. tocopherols. and other antioxidants, are frequently added to drug formulations specifically to decrease oxidation. Therefore, since our invention specifically pertains to a formulation containing omega-3, omega-6, or omega-9 polyunsaturated fatty acids, for the purposes of this invention the antioxidant levels will be limited only by the antioxidant activity of the antioxidant added to the formulation and/or the maximum tolerated dose.

The therapeutic procedures and compositions are here described with reference to humans, however they may also be employed in the field of veterinary medicine particularly in the treatment of domestic animals and commercial livestock.

For purposes of illustration, considering budesonide as the anti-inflammatory drug employed, dosages may vary up to 9 mg per day (recommended daily dose) in single or divided doses but are usually in the range of 3 mg per dose up to 3 doses per day. The maximum tolerated dose is unknown, and the maximum dose used would be dependent upon the comparison of efficacy and adverse manifestations by a medical professional, See also U.S. 5,643,602 for a listing of illustrative anti-inflammatory steroids, dosages and other information.

The compositions of the invention include various presentations and formulations. For example all three "components" may be included in a single unit dosage form such as a capsule or as a liquid for oral ingestion or topical application for esophageal or stomach indications. Solid oral dosage forms will be formulated for delivery to the desired site of treatment such as by enteric coating tablets or capsules with a pH soluble-dependent coating. Topical and ophthalmic formulations, transdermal patches, solution/suspensions/emulsions are also within the scope of the invention.

The essential components of the therapeutic combination may be administered separately at different times or simultaneously at the same time, sequentially or together conveniently in a single unit dosage.

The formulations of the present invention are appropriate for administration by any convenient route, including, but not limited to the following: oral, enteral (tube-fed), rectal, ophthalmic, dermatologic, subcutaneous, intramuscular, intraperitoneal, or intravenous.

Preferably the relative proportions of drug and omega-3 are such that the daily dose of omega-3 is from 0.1 to 30.0 grams per day and the relative proportions of the tocopherols and other active ingredients are such that the daily dose of tocopherols is from 1 to 1000 IU per day.

The components may be solubilized in an oil vehicle such as the omega-3 polyunsaturated fatty acids source.

The invention includes several embodiments, such as illustrated by but not limited to the following: In one embodiment a composition is from a source of omega-3 and/or omega-6 and/or omega-9, a source of antioxidants, e.g. tocopherols and a drug, e.g. budesonide.

In an embodiment the lipid component is fish (marine) oil, or fractions thereof which are relatively enriched in omega-3. A source of antioxidants such as tocopherols (e.g. alpha- and/or gamma-tocopherol) is added to the omega-3.

In an embodiment the lipid component is derived from a plant source, which is used for its high content of omega-3 or omega-6 or omega-9.

In an embodiment the lipid component is a synthesized, purified, or enriched source of the omega-3, omega-6, or omega-9 polyunsaturated fatty acids themselves (e.g. EPA, DHA for omega-3) which may be in the form of tri-, di- or mono-glycerides, methyl/ethyl esters, free fatty acids or other similar bioavailable forms.

The lipid component may be provided in the form of a purified oil having at least 50% of its lipid content comprising either omega-3 and/or omega-6 and/or omega-9 polyunsaturated fatty acids.

In an embodiment, the invention formulation contains, as active ingredients: a source of polyunsaturated acids (omega-3 and/or omega-6 and/or omega-9; a drug (e.g. budesonide); and an antioxidant such as tocopherols (e.g. alpha- and/or gamma tocopherols). Other, similar drugs may also be employed, in place of or in conjunction with budesonide.

Since the invention is intended for treating inflammation of the GIT, as well as systemic and local inflammation multiple formulations are preferred, but not essential to the invention. Multiple formulations means a number of preparations which differ in the ratio of polyunsaturated acid to drug. For example, the ratio of omega-3 to antioxidant is preferred to be constant, as a function of stability considerations, although the ratio could be varied. This allows the tapering off of the drug component of the therapeutic combination, while maintaining a constant dose of nutrients (omega-3, tocopherols). Further, since patients respond differently, and the same patient may respond differently at different times, the "optimal" dose (i.e. ratio omega-3: drug) may vary. This may initially have to be determined on a case-by-case basis. Multiple formulations allow selection of tailored dosing to accommodate this situation.

Another strategy for multiple formations involves keeping the ratio omega-3: drug constant, but reducing the total dose over time. Multiple reduced dosing allows the patient to continue taking the same number of medications or unit dosage forms such as capsules or the like daily, in a "tapering" fashion. In addition, it should be noted that for formulations intended for release of formulation at specific intestinal targets, the type of oil used could be modified or chosen to provide an oil that would be rapidly, or less rapidly, absorbed, thereby providing drug for immediate, or delayed, uptake.

The medicament prepared according to the invention may be administered daily for at least two weeks, and thereafter treatment continues either daily or every other day. For example, after the first two weeks. administration is either daily or every other day and the amount of anti-inflammatory drug is gradually reduced over time as the patient's symptoms decrease while the amount of omega-3 and/or omega-6 and/or omega-9 polyunsaturated fatty acids and antioxidants remains substantially constant. Alternatively, after the first two weeks, administration is either daily or every other day and the amount of anti-inflammatory drug and omega-3 and/or omega-6 and /or omega -9 polyunsaturated fatty acid and antioxidants are proportionally reduced or increased over time as the patient's symptoms change.

Alternatively, the medicament may be administered every other day for at least two weeks, and thereafter treatment continues either daily or every other day.

The invention further provides an orally or enterally administrable composition for the treatment of inflammatory conditions of the gastrointestinal tract consisting essentially of effective amounts of budesonide, or its prodrugs or derivatives, a highly purified oil source of omega-3 and/or omega-6 and/or omega-9 polyunsaturated fatty acids, and antioxidants, optionally together with pharmaceutically acceptable carriers, diluents, viscosity-modifiers, stabilizers, and erodable or swallowable matrices.

The invention further provides an orally administrable composition for the treatment of systemic or local inflammatory conditions consisting essentially of effective amounts of budesonide, or its prodrugs or derivatives, a highly purified oil source of omega-3 and/or omega-6 and/or omega-9 polyunsaturated fatty acids, and antioxidants, optionally including pharmaceutically acceptable carriers, diluents, viscosity-modifiers, stabilizers, erodable or swallowable matrices, and penetration enhancers.

In the above-described orally or enterally administrable composition, the composition may be encapsulated and enteric-coated for release and/or delivery of drug of about 55% into the proximal small bowel. Alternatively, the composition may be encapsulated and enteric-coated for release and/or delivery of drug of about 25% into the distal small bowel. As a further alternative, the composition may be encapsulated and enteric-coated for release and/or delivery of drug of about 25% into the ascending and transverse colon.

The invention further provides a topically administrable composition for the treatment of systemic or local inflammatory conditions consisting essentially of effective amounts of budesonide, or its prodrugs or derivatives, omega-3 and/or omega-6 and/or omega-9 polyunsaturated fatty acids and antioxidants, optionally including pharmaceutically acceptable carriers, diluents, viscosity-modifiers, stabilizers, erodable matrices, and penetration enhancers.

The present invention is further illustrated in the following general formulations related to the locus of the inflammatory condition to be treated.
- Type I:: Encapsulated fully solubilized budesonide in free fatty acids with high or low viscosity, enteric-coated for delivery to proximal small bowel.
- Upon release:: Immediate uptake of oil and budesonide with no effects on formulation spread.
- Disease location:: Systemic inflammation.
- Type 2:: Encapsulated fully suspended budesonide in triglyceride fatty acids with high or low viscosity, enteric-coated for delivery to proximal small bowel.
- Upon release:: Immediate uptake of oil and delayed uptake of budesonide, some formulation spread
- Disease location:: Systemic inflammation or proximal small bowel.
- Type 3:: Encapsulated fully solubilized budesonide in triglyceride mix with high or low viscosity, enteric-coated for delivery to distal small bowel.
- Upon release:: Some delay in uptake of oil, some immediate uptake of budesonide, formulation spreading, and minimal changes in residence time.
- Disease location:: Distal small bowel disease and ascending colon.
- Type 4:: Encapsulated fully suspended budesonide in triglyceride fatty acids with high viscosity, enteric-coated for delivery to distal small bowel.
- Uptake:: Some delay in uptake of oil, delay in uptake of budesonide. maximal formulation spread, and some increase in residence time.
- Disease location:: Distal small bowel, ascending and transverse.
- Type 5:: Encapsulated fully solubilized budesonide in free fatty acids with low viscosity, enteric-coated for delivery to ascending colon.
- Uptake:: Immediate uptake of oil and budesonide, formulation spread, and short residence time.
- Disease location:: Ascending and transverse colon.
- Type 6:: Encapsulated fully suspended budesonide in triglycerides with high viscosity enteric-coated for delivery to ascending colon.
- Uptake:: Delayed uptake of oil and budesonide, maximal formulation spread, and long residence time.
- Disease location:: Ascending and transverse colon.

It is understood that the application of the teachings of the present invention, to the conditions described, will be evident to one skilled in the art of preparing such formulations, and to one skilled in treating such medical conditions.

Additional features and advantages of the present invention are described below in preferred embodiments, which are intended as example, and not as limitation.

### Preferred Embodiments

### Example #1:

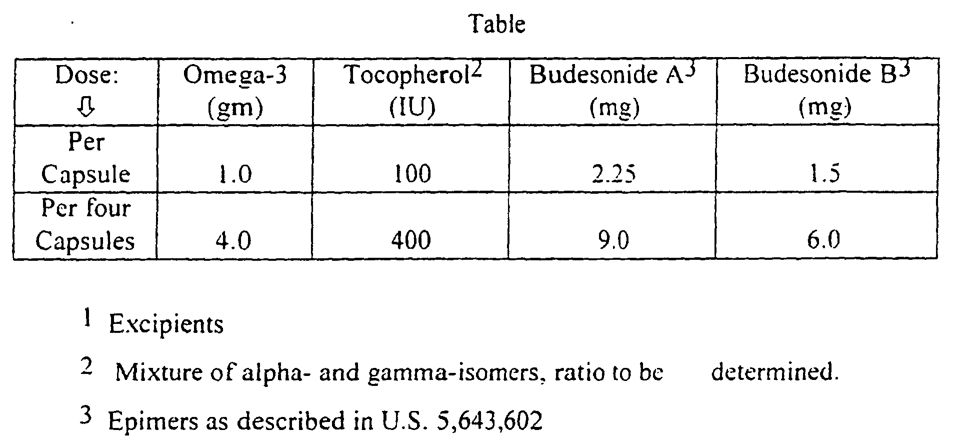

The following contemplated clinical cases of Crohn's disease are presented as examples of methods of treatment for IBD, and not as limitations. Other illustrations of treating systemic or localized inflammatory reactions are not presented, since the principles involved are similar, and will be apparent to the skilled reader based upon review of an individual's clinical manifestation and the preferred embodiments of the inventive compositions.

### Example #1.

Two adults present in the emergency room of a hospital. Each is determined to be experiencing an acute relapse of Crohn's disease. Each has a history of CD over the past 5 years, with an acute episode about every 8-10 months. Patient #1 has CDAI score of 410. indicating a severe flare-up (CDAI is a commonly used index of disease severity; score value is directly related to severity). He is started on budesonide. 9mg/day, twice daily. The dose is scheduled to be tapered off to 6, then 3 mg over the next 8-10 weeks, if the condition improves sufficiently.

Patient #2 has a CDAI score of 450, at least as severe as that of patient #1. This patient is also started on a formulation of 9 mg/day of budesonide, but formulated as the omega-3/tocopherol version (see Table). Tapering was also planned, over an 8 week period. Neither subject knew which formulation was being taken. Both subjects began consuming 100% of their nutrition from a simple medical food that was not "elemental" in its content, and contained small amounts of omega-3.

Patient #1 (budesonide) showed a gradual drop in CDAI, from 410 at baseline, to 290 at week 2; to 220 at week four; with gradual stabilization at 145 by 6 weeks. Budesonide was lowered to 6 mg/day at week 8; and to 3 mg/day; it was discontinued at week 12. The patient was able to consume a highly restricted diet, by 6 weeks. He graduated to "bland", low residue foods, at week 10, and to "normal" foods at week 12. He was judged as fully recovered at week 14.

Patient #2 (budesonide + omega-3) showed a drop in CDAI from 450 at baseline, to 220 at week two; and a further drop to 170 at week 4; stabilizing at 120 by week 6. This patient began consuming a "bland", low residue diet by week 6, and normal foods by week 8-9. He was judged fully recovered at week 10.

### Example II.

Two patients with CD, in remission for the last 10 months, were screened and found to be at high risk of relapse. This was judged on the basis of elevated biochemical markers of inflammation, namely, C-Reactive Protein (CRP). alpha- acid glycoprotein, and erythrocyte sedimentation rate (ESR). Patient #1 began receiving a "prophylactic" dose of budesonide, 2 mg/day, BID. Patient #2 began receiving a formulation of budesonide in a daily dose of 1.0 mg/day; omega-3. in a dose of 3.0 grams/day; and tocopherol in a dose of 75 IU/day. Both patients also happened to manifest extra-intestinal (systemic) symptoms, including arthritis and conjunctivitis. Neither patient. nor physician. knew the identity of their prescription. Each formulation was intended to be continued until a relapse occurred. or for one year.

Patient #1 (budesonide) showed increases in the values of the inflammatory markers at month 2, a greater increase at month 4. Shortly after the 4-month blood sample was analyzed, the patient began experiencing worsening clinical symptoms of GIT inflammation, and was judged to be in full relapse before month 5 was reached. She was admitted to hospital for stabilization, and began receiving the preparation containing budesonide + omega-3; and went into remission by week 8 of this treatment. Patient's systemic symptoms did not change.
Patient #2 showed stable biochemical risk factors at month 2, a noticeable drop at month 4, and fully normal values by month 6. The patient had not relapsed when the study treatment was terminated at month 12. Subject's systemic symptoms were significantly improved in less than 6 months. Subject was weaned from budesonide at this time, but elected to continue taking commercial preparations of omega-3 thereafter.

## Claims

1. A pharmaceutical composition for the treatment of inflammatory conditions in mammals, said composition consisting essentially of:
budesonide or a prodrug or derivative thereof,
a polyunsaturated fatty acid comprising one or more of omega-3, omega-6, and omega-9, or a derivative thereof,
a pharmacologically active antioxidant, and optionally
a pharmaceutically acceptable carrier including one or more of other essential oils, non-essential oils, diluents, viscosity-modifiers, stabilizers, erodable matrices, swallowable matrices, and penetration enhancers.

2. The pharmaceutical composition of claim 1, consisting of:
budesonide,
a polyunsaturated fatty acid comprising one or more of omega-3, omega-6, and omega-9,
a pharmacologically active antioxidant, and
a pharmaceutically acceptable carrier including one or more of other essential oils, non-essential oils, diluents, viscosity-modifiers, stabilizers, erodable matrices, swallowable matrices, and penetration enhancers.

3. The pharmaceutical composition of claim 1 or 2, wherein the pharmacologically active antioxidant comprises tocopherol.

4. The pharmaceutical composition of claim 1 or 2, wherein the pharmacologically active antioxidant comprises a mixture of alpha- and gamma-isomers of tocopherol.

5. The pharmaceutical composition of any one of claims 1-4, wherein the polyunsaturated fatty acid is a purified oil having at least 50% of a lipid content comprising either omega-3 and/or omega-6 and/or omega-9 polyunsaturated fatty acid.

6. The pharmaceutical composition of any one of claims 1-5, wherein the omega-3, omega-6, omega-9 polyunsaturated fatty acid, or a derivative thereof, is in a bioavailable form.

7. The pharmaceutical composition of claim 6, wherein the bioavailable form is a tri-, di-, or mono-glyceride, methyl/ethyl ester, or free fatty acid.

8. The pharmaceutical composition of any one of claims 1-7, wherein the budesonide, or a prodrug or derivative thereof, is fully solubilized.

9. The pharmaceutical composition of any one of claims 1-7, wherein the budesonide, or a prodrug or derivative thereof, is fully suspended.

10. The pharmaceutical composition of any one of claims 1-7, wherein the budesonide, or a prodrug or derivative thereof, is partially suspended.

11. The pharmaceutical composition of any one of claims 1-10, wherein the composition provides from 0.1 to 30 grams of omega-3 and/or omega-6 and/or omega-9 polyunsaturated fatty acid, or a derivative thereof.

12. A pharmaceutical composition for the treatment of inflammatory conditions in mammals comprising:
omega-3 polyunsaturated fatty acid, tocopherol, budesonide A, and budesonide B.

13. The pharmaceutical composition of claim 12, comprising
1.0 gram omega-3 polyunsaturated fatty acid,
100 IU tocopherol,
2.25 milligrams budesonide A, and
1.5 milligrams budesonide B.

14. An orally or enterally administrable composition for use in the treatment of inflammatory conditions of the gastrointestinal tract comprising effective amounts of active ingredients comprising budesonide, a prodrug or derivative thereof, a highly purified oil source of omega-3 and/or omega-6 and/or omega-9 polyunsaturated fatty acid or a derivative thereof, and an antioxidant, optionally also including one or more of pharmaceutically acceptable carriers, diluents, viscosity-modifiers, stabilizers, erodable matrices, swallowable matrices, and penetration enhancers.

15. An orally administrable composition for use in the treatment of systemic or inflammatory conditions comprising effective amounts of active ingredients comprising budesonide, or a prodrug or derivative thereof, a highly purified oil source of omega-3 and/or omega-6 and/or omega-9 polyunsaturated fatty acid, or a derivative thereof, and a pharmacologically active antioxidant, optionally also including one or more of a pharmaceutically acceptable carrier, including other essential and non-essential oils, diluents, viscosity-modifiers, stabilizers, erodable matrices, swallowable matrices, and penetration enhancers.

16. The composition of claim 14 or 15, wherein the antioxidant is a tocopherol.

17. The composition of claim 16, wherein the tocopherol is a mixture of alpha- and gamma-isomers.

18. The composition of claim 14 or 15, wherein the composition is encapsulated and enteric-coated for release and/or delivery of budesonide of about 55% into the proximal small bowel.

19. The composition of claim 14 or 15, wherein said composition is encapsulated and enteric-coated for release and/or delivery of budesonide of about 25% into the distal small bowel.

20. The composition of claim 14 or 15, wherein said composition is encapsulated and enteric-coated for release and/or delivery of budesonide of about 25% into the ascending and transverse colon.

21. A topically administrable composition for the treatment of systemic or local inflammatory conditions comprising effective amounts of budesonide, or a prodrug or derivative thereof, omega-3 and/or omega-6 and/or omega-9 polyunsaturated fatty acid or a derivative thereof, and an antioxidant, optionally including one or more of pharmaceutically acceptable carriers, diluents, viscosity-modifiers, stabilizers, erodable matrices, and penetration enhancers.

22. Use of the composition of any one of the preceding claims in the preparation of a medicament for treating inflammation of the gastrointestinal tract and/or systemic or local inflammation in an animal.

23. Use of claim 22, wherein the composition is adapted for administration to a luminal side of the gastrointestinal tract.

24. Use of the composition of any one of claims 1-21, wherein budesonide or a prodrug or derivative thereof and the polyunsaturated fatty acid comprising one or more of omega-3, omega-6, and omega-9, or a derivative thereof are administered simultaneously.

25. Use of the composition of any one of claims 1-21, wherein budesonide or a prodrug or derivative thereof, the polyunsaturated fatty acid comprising one or more of omega-3, omega-6, and omega-9, or a derivative thereof, and the antioxidant are administered simultaneously.

26. A kit of parts consisting essentially of:
budesonide or a prodrug or derivative thereof,
a polyunsaturated fatty acid comprising one or more of omega-3, omega-6, and omega-9, or a derivative thereof, and
a pharmacologically active antioxidant.

27. The kit of parts of claim 26, further comprising a pharmaceutically acceptable carrier including one or more of other essential oils, non-essential oils, diluents, viscosity-modifiers, stabilizers, erodable matrices, swallowable matrices, and penetration enhancers.

28. The kit of parts of claim 26 or 27, wherein the pharmacologically active antioxidant comprises tocopherol.

29. The kit of parts of claim 26 or 27, wherein the pharmacologically active antioxidant comprises a mixture of alpha- and gamma-isomers of tocopherol.

30. The kit of parts of any one of claims 26-29, wherein the polyunsaturated fatty acid is a purified oil having at least 50% of a lipid content comprising either omega-3 and/or omega-6 and/or omega-9 polyunsaturated fatty acid.

31. The kit of parts of any one of claims 26-30, wherein the omega-3, omega-6, omega-9 polyunsaturated fatty acid, or a derivative thereof, is in a bioavailable form.

32. The kit of parts of claim 31, wherein the bioavailable form is a tri-, di-, or mono-glyceride, methyl/ethyl ester, or free fatty acid.

33. The kit of parts of any one of claims 26-32, wherein the budesonide, or a prodrug or derivative thereof, is fully solubilized.

34. The kit of parts of any one of claims 26-32, wherein the budesonide, or a prodrug or derivative thereof, is fully suspended.

35. The kit of parts of any one of claims 26-32, wherein the budesonide, or a prodrug or derivative thereof, is partially suspended.

36. The kit of parts of any one of claims 26-35, wherein the composition provides from 0.1 to 30 grams of omega-3 and/or omega-6 and/or omega-9 polyunsaturated fatty acid, or a derivative thereof.

37. The kit of parts of any one of claims 26-36, wherein the kit of parts is orally, enterally, or topically administrable.

38. The kit of parts of any one of claims 26-37, wherein the kit of parts is adapted for simultaneous administration.

## Patentansprüche

1. Pharmazeutische Zusammensetzung zur Behandlung von entzündlichen Zuständen bei Säugetieren, wobei die Zusammensetzung im Wesentlichen besteht aus:
Budesonid oder einem Pro-Arzneimittel oder einem Derivat davon,
einer mehrfach ungesättigten Fettsäure, die eines oder mehrere von Omega-3, Omega-6 und Omega-9 oder ein Derivat davon enthält,
einem pharmakologisch aktives Antioxidans und gegebenenfalls
einem pharmazeutisch akzeptablen Träger, der eines oder mehrere von zusätzlich ätherischen Ölen, nicht-ätherischen Ölen, Verdünnungsmitteln, Viskositätsverbesserern, Stabilisatoren, erodierbaren Matrizen, schluckbaren Matrizen und Penetrationsverstärkern enthält.

2. Pharmazeutische Zusammensetzung nach Anspruch 1, bestehend aus:
Budesonid,
einer mehrfach ungesättigten Fettsäure, die eines oder mehrere von Omega-3, Omega-6 und Omega-9 enthält,
einem pharmakologisch aktives Antioxidans und
einem pharmazeutisch akzeptablen Träger, der eines oder mehrere von zusätzlich ätherischen Ölen, nicht-ätherischen Ölen, Verdünnungsmitteln, Viskositätsverbesserern, Stabilisatoren, erodierbaren Matrizen, schluckbaren Matrizen und Penetrationsverstärkern enthält.

3. Pharmazeutische Zusammensetzung nach Anspruch 1 oder 2, wobei das pharmakologisch aktive Antioxidans Tocopherol aufweist.

4. Pharmazeutische Zusammensetzung nach Anspruch 1 oder 2, wobei das pharmakologisch aktive Antioxidans eine Mischung aus Alpha- und Gamma-Isomeren von Tocopherol aufweist.

5. Pharmazeutische Zusammensetzung nach einem der Ansprüche 1 bis 4, wobei die mehrfach ungesättigte Fettsäure ein gereinigtes Öl ist, das mindestens 50 % eines Lipidgehalts aufweist, der entweder Omega-3 und/oder Omega-6 und/oder Omega-9 mehrfach ungesättigte Fettsäure enthält.

6. Pharmazeutische Zusammensetzung nach einem der Ansprüche 1 bis 4, wobei die Omega-3, Omega-6, Omega-9 mehrfach ungesättigte Fettsäure oder ein Derivat davon in einer biologisch verfügbaren Form vorliegt.

7. Pharmazeutische Zusammensetzung nach Anspruch 6, wobei die biologisch verfügbare Form ein Tri-, Di-, oder Monoglycerid, Methyl-/Ethylester oder eine freie Fettsäure ist.

8. Pharmazeutische Zusammensetzung nach einem der Ansprüche 1 bis 7, wobei das Budesonid oder ein Pro-Arzneimittel oder ein Derivat davon vollständig löslich gemacht ist.

9. Pharmazeutische Zusammensetzung nach einem der Ansprüche 1 bis 7, wobei das Budesonid oder ein Pro-Arzneimittel oder ein Derivat davon vollständig suspendiert ist.

10. Pharmazeutische Zusammensetzung nach einem der Ansprüche 1 bis 7, wobei das Budesonid oder ein Pro-Arzneimittel oder ein Derivat davon teilweise suspendiert ist.

11. Pharmazeutische Zusammensetzung nach einem der Ansprüche 1 bis 10, wobei die Zusammensetzung von 0,1 bis 30 Gramm Omega-3 und/oder Omega-6 und/oder Omega-9 mehrfach ungesättigte Fettsäure oder ein Derivat davon bereitstellt.

12. Pharmazeutische Zusammensetzung zur Behandlung von entzündlichen Zuständen bei Säugetieren, welche enthält:
Omega-3 mehrfach ungesättigte Fettsäure, Tocopherol, Budesonid A und Budesonid B.

13. Pharmazeutische Zusammensetzung nach Anspruch 12, welche enthält:
1,0 Gramm Omega-3 mehrfach ungesättigte Fettsäure,
100 IU Tocopherol,
2,25 Milligramm Budesonid A und
1,5 Milligramm Budesonid B.

14. Oral oder enteral verabreichbare Zusammensetzung zur Verwendung bei der Behandlung von entzündlichen Zuständen des Magen-Darm-Traktes, die wirksame Mengen von aktiven Inhaltsstoffen enthält, welche Budesonid, ein Pro-Arzneimittel oder Derivat davon, eine hoch gereinigte Ölquelle aus Omega-3 und/oder Omega-6 und/oder Omega-9 mehrfach ungesättigter Fettsäure oder einem Derivat davon und ein Antioxidans enthält, gegebenenfalls auch einschließlich von einem oder mehreren von pharmazeutisch akzeptablen Trägern, Verdünnungsmitteln, Viskositätsverbesserern, Stabilisatoren, erodierbaren Matrizen, schluckbaren Matrizen und Penetrationsverstärkern.

15. Oral verabreichbare Zusammensetzung zur Verwendung bei der Behandlung von systemischen oder entzündlichen Zuständen, die wirksame Mengen an aktiven Inhaltsstoffen enthält, welche Budesonid oder ein Pro-Arzneimittel oder Derivat davon, eine hoch gereinigte Ölquelle aus Omega-3 und/oder Omega-6 und/oder Omega-9 mehrfach ungesättigter Fettsäure oder ein Derivat davon und ein pharmakologisch aktives Antioxidans enthält, gegebenenfalls auch einschließlich von einem oder mehreren aus einem pharmazeutisch akzeptablen Träger, einschließlich zusätzlicher ätherischer und nicht-ätherischer Öle, Verdünnungsmittel, Viskositätsverbesserern, Stabilisatoren, erodierbaren Matrizen, schluckbaren Matrizen und Penetrationsverstärkern.

16. Zusammensetzung nach Anspruch 14 oder 15, wobei das Antioxidans ein Tocopherol ist.

17. Zusammensetzung nach Anspruch 16, wobei das Tocopherol eine Mischung aus Alpha- und Gamma-Isomeren ist.

18. Zusammensetzung nach Anspruch 14 oder 15, wobei die Zusammensetzung zur Freisetzung und/oder Zufuhr von ungefähr 55 % Budesonid in den proximalen Dünndarm eingekapselt und mit einer säureresistenten Schicht überzogen ist.

19. Zusammensetzung nach Anspruch 14 oder 15, wobei die Zusammensetzung zur Freisetzung und/oder Zufuhr von ungefähr 25 % Budesonid in den distalen Dünndarm eingekapselt und mit einer säureresistenten Schicht überzogen ist.

20. Zusammensetzung nach Anspruch 14 oder 15, wobei die Zusammensetzung zur Freisetzung und/oder Zufuhr von ungefähr 25 % Budesonid in das aufsteigende Colon und das Quercolon eingekapselt und mit einer säureresistenten Schicht überzogen ist.

21. Lokal verabreichbare Zusammensetzung zur Behandlung von systemischen oder lokalen entzündlichen Zuständen, welche wirksame Mengen von Budesonid oder einem Pro-Arzneimittel oder Derivat davon, Omega-3 und/oder Omega-6 und/oder Omega-9 mehrfach ungesättigte Fettsäure oder ein Derivat davon und ein Antioxidans umfasst, optional einschließlich einem oder mehreren von pharmazeutisch akzeptablen Trägern, Verdünnungsmitteln, Viskositätsverbesserern, Stabilisatoren, erodierbaren Matrizen und Penetrationsverstärkern.

22. Verwendung der Zusammensetzung nach einem der vorhergehenden Ansprüche bei der Herstellung eines Medikamentes zur Behandlung einer Entzündung des Magen-Darm-Traktes und/oder einer systemischen oder lokalen Entzündung in einem Tier.

23. Verwendung nach Anspruch 22, wobei die Zusammensetzung zur Verabreichung an eine luminale Seite des Magen-Darm-Traktes geeignet ist.

24. Verwendung der Zusammensetzung nach einem der Ansprüche 1 bis 21, wobei Budesonid oder ein Pro-Arzneimittel oder Derivat davon und die mehrfach ungesättigte Fettsäure, welche eines oder mehrere von Omega-3, Omega-6 und Omega-9 oder ein Derivat davon enthält, gleichzeitig verabreicht werden.

25. Verwendung der Zusammensetzung nach einem der Ansprüche 1 bis 21, wobei Budesonid oder ein Pro-Arzneimittel oder Derivat davon, die mehrfach ungesättigte Fettsäure, welche eines oder mehrere von Omega-3, Omega-6 und Omega-9 oder ein Derivat davon enthält, und das Antioxidans gleichzeitig verabreicht werden.

26. Satz aus Teilen, der im Wesentlichen besteht aus:
Budesonid oder einem Pro-Arzneimittel oder Derivat davon,
einer mehrfach ungesättigten Fettsäure, welche eines oder mehrere von Omega-3, Omega-6 und Omega-9 oder ein Derivat davon enthält, und
einem pharmakologisch aktiven Antioxidans.

27. Satz aus Teilen nach Anspruch 26, der femer einen pharmazeutisch akzeptablen Träger enthält, der ein oder mehrere von zusätzlich ätherischen Ölen, nicht-ätherischen Ölen, Verdünnungsmitteln, Viskositätsverbessern, Stabilisatoren, erodierbaren Matrizen, schluckbaren Matrizen und Penetrationsverstärkern enthält.

28. Satz aus Teilen nach Anspruch 26 oder 27, wobei das pharmakologisch aktive Antioxidans Tocopherol aufweist.

29. Satz aus Teilen nach Anspruch 26 oder 27, wobei das pharmakologisch aktive Antioxidans eine Mischung aus Alpha- und Gamma-Isomeren von Tocopherol enthält.

30. Satz aus Teilen nach einem der Ansprüche 26 bis 29, wobei die mehrfach ungesättigte Fettsäure ein gereinigtes Öl ist, das mindestens 50 % eines Lipidgehalts aufweist, der entweder Omega-3 und/oder Omega-6 und/oder Omega-9 mehrfach ungesättigte Fettsäure enthält.

31. Satz an Teilen nach einem der Ansprüche 26 - 30, wobei die Omega-3, Omega-6, Omega-9 mehrfach ungesättigte Fettsäure oder ein Derivat davon in biologisch verfügbarer Form vorliegt.

32. Satz aus Teilen nach Anspruch 31, wobei die biologisch verfügbare Form ein Tri-, Di- oder Monoglycerid, Methyl-/Ethylester oder eine freie Fettsäure ist.

33. Satz aus Teilen nach einem der Ansprüche 26 bis 32, wobei das Budesonid oder ein Pro-Arzneimittel oder Derivat davon vollständig löslich gemacht ist.

34. Satz aus Teilen nach einem der Ansprüche 26 bis 32, wobei das Budesonid oder ein Pro-Arzneimittel oder ein Derivat davon vollständig suspendiert ist.

35. Satz aus Teilen nach einem der Ansprüche 26 bis 32, wobei das Budesonid oder ein Pro-Arzneimittel oder Derivat davon teilweise suspendiert ist.

36. Satz aus Teilen nach einem der Ansprüche 26 bis 35, wobei die Zusammensetzung von 0,1 bis 30 Gramm Omega-3 und/oder Omega-6 und/oder Omega-9 mehrfach ungesättigte Fettsäure oder ein Derivat davon bereitstellt.

37. Satz aus Teilen nach einem der Ansprüche 26 bis 36, wobei der Satz aus Teilen oral, enteral oder lokal verabreichbar ist.

38. Satz aus Teilen nach einem der Ansprüche 26 bis 37, wobei der Satz aus Teilen für gleichzeitige Verabreichung geeignet ist.

## Revendications

1. Composition pharmaceutique pour le traitement d'états inflammatoires chez des mammifères, ladite composition comprenant essentiellement :
du budesonide ou un promédicament ou dérivé de celui-ci,
un acide gras polyinsaturé comprenant un ou plusieurs parmi l'oméga-3, l'oméga-6 et l'oméga-9, ou un dérivé de ceux-ci,
un antioxydant pharmacologiquement actif, et facultativement
un vecteur pharmaceutiquement acceptable comprenant un ou plusieurs parmi d'autres huiles essentielles, des huiles non essentielles, des diluants, des modificateurs de viscosité, des stabilisants, des matrices pouvant être érodées, des matrices pouvant être avalées, et des agents améliorant la pénétration.

2. Composition pharmaceutique suivant la revendication 1, comprenant
du budesonide,
un acide gras polyinsaturé comprenant un ou plusieurs parmi l'oméga-3, l'oméga-6 et l'oméga-9,
un antioxydant pharmacologiquement actif, et
un vecteur pharmaceutiquement acceptable comprenant un ou plusieurs parmi d'autres huiles essentielles, des huiles non essentielles, des diluants, des modificateurs de viscosité, des stabilisants, des matrices pouvant être érodées, des matrices pouvant être avalées, et des agents améliorant la pénétration.

3. Composition pharmaceutique suivant la revendication 1 ou 2, dans laquelle l'antioxydant pharmacologiquement actif comprend du tocophérol.

4. Composition pharmaceutique suivant la revendication 1 ou 2, dans laquelle l'antioxydant pharmacologiquement actif comprend un mélange des isomères alpha et gamma du tocophérol.

5. Composition pharmaceutique suivant l'une quelconque des revendications 1-4, dans laquelle l'acide gras polyinsaturé est une huile purifiée présentant au moins 50% de sa teneur en lipides comprenant un acide gras polyinsaturé oméga-3 et/ou oméga-6 et/ou oméga-9.

6. Composition pharmaceutique suivant l'une quelconque des revendications 1-5, dans laquelle l'acide gras polyinsaturé oméga-3, oméga-6, oméga-9 ou un dérivé de ceux-ci est sous une forme biodisponible.

7. Composition pharmaceutique suivant la revendication 6, dans laquelle la forme biodisponible est un tri-, un di- ou un monoglycéride, un méthyl/éthylester ou un acide gras libre.

8. Composition pharmaceutique suivant l'une quelconque des revendications 1-7, dans laquelle le budesonide ou un promédicament ou un dérivé de celui-ci est entièrement en solution.

9. Composition pharmaceutique suivant l'une quelconque des revendications 1-7, dans laquelle le budesonide ou un promédicament ou un dérivé de celui-ci est entièrement en suspension.

10. Composition pharmaceutique suivant l'une quelconque des revendications 1-7, dans laquelle le budesonide ou un promédicament ou un dérivé de celui-ci est partiellement en suspension.

11. Composition pharmaceutique suivant l'une quelconque des revendications 1-10, dans laquelle la composition procure de 0,1 à 30 grammes d'acide gras polyinsaturé oméga-3 et/ou oméga-6 et/ou oméga-9 ou d'un dérivé de ceux-ci.

12. Composition pharmaceutique pour le traitement d'états inflammatoires chez des mammifères, comprenant :
de l'acide gras polyinsaturé oméga-3, du tocophérol, du budesonide A et du budesonide B.

13. Composition pharmaceutique suivant la revendication 12, comprenant :
1,0 gramme d'acide gras polyinsaturé oméga-3,
100 UI de tocophérol,
2,25 milligrammes de budesonide A, et
1,5 milligramme de budesonide B.

14. Composition pouvant être administrée par voie orale ou entérale pour une utilisation dans le traitement d'états inflammatoires du tractus gastro-intestinal comprenant des quantités efficaces d'ingrédients actifs comprenant du budesonide, un promédicament ou un dérivé de celui-ci, une source d'huile hautement purifiée d'acide gras polyinsaturé oméga-3 et/ou oméga-6 et/ou oméga-9 ou d'un dérivé de ceux-ci et un antioxydant, comprenant également facultativement un ou plusieurs vecteurs, diluants, modificateurs de la viscosité, stabilisants, matrices pouvant être érodées, matrices pouvant être avalées, et agents améliorant la pénétration pharmaceutiquement acceptables.

15. Composition pouvant être administrée par voie orale pour une utilisation dans le traitement d'états systémiques ou inflammatoires comprenant des quantités efficaces d'ingrédients actifs comprenant du budesonide, ou un promédicament ou un dérivé de celui-ci, une source d'huile hautement purifiée d'acide gras polyinsaturé oméga-3 et/ou oméga-6 et/ou oméga-9 ou d'un dérivé de ceux-ci et un antioxydant pharmacologiquement actif, comprenant également facultativement un ou plusieurs parmi un vecteur pharmaceutiquement acceptable, notamment d'autres huiles essentielles et non essentielles, des diluants, des modificateurs de la viscosité, des stabilisants, des matrices pouvant être érodées, des matrices pouvant être avalées, et des agents améliorant la pénétration.

16. Composition suivant la revendication 14 ou 15, dans laquelle l'antioxydant est un tocophérol.

17. Composition suivant la revendication 16, dans laquelle le tocophérol est un mélange d'isomères alpha et gamma.

18. Composition suivant la revendication 14 ou 15, dans laquelle la composition est enrobée et recouverte d'un revêtement entérique pour une libération et/ou une délivrance du budesonide pour environ 55% dans l'intestin grêle proximal.

19. Composition suivant la revendication 14 ou 15, dans laquelle ladite composition est enrobée et recouverte d'un revêtement entérique pour une libération et/ou une délivrance du budesonide pour environ 25% dans l'intestin grêle distal.

20. Composition suivant la revendication 14 ou 15, dans laquelle ladite composition est enrobée et recouverte d'un revêtement entérique pour une libération et/ou une délivrance du budesonide pour environ 25% dans le côlon ascendant et moyen.

21. Composition pouvant être administrée par voie topique pour le traitement d'états inflammatoires systémiques ou locaux comprenant des quantités efficaces de budesonide, ou d'un promédicament ou d'un dérivé de celui-ci, un acide gras polyinsaturé oméga-3 et/ou oméga-6 et/ou oméga-9 ou un dérivé de ceux-ci et un antioxydant, comprenant également facultativement un ou plusieurs parmi des vecteurs, des diluants, des modificateurs de la viscosité, des stabilisants, des matrices pouvant être érodées, et des agents améliorant la pénétration pharmaceutiquement acceptables.

22. Utilisation de la composition suivant l'une quelconque des revendications précédentes dans la préparation d'un médicament pour le traitement d'une inflammation du tractus gastro-intestinal et/ou d'une inflammation systémique ou locale chez un animal.

23. Utilisation suivant la revendication 22, dans laquelle la composition est adaptée pour une administration du côté d'une lumière du tractus gastro-intestinal.

24. Utilisation de la composition suivant l'une quelconque des revendications 1-21, dans laquelle le budesonide ou un promédicament ou un dérivé de celui-ci et l'acide gras polyinsaturé comprenant un ou plusieurs parmi l'oméga-3, l'oméga-6 et l'oméga-9 ou un dérivé de ceux-ci sont administrés de manière simultanée.

25. Utilisation de la composition suivant l'une quelconque des revendications 1-21, dans laquelle le budesonide ou un promédicament ou un dérivé de celui-ci et l'acide gras polyinsaturé comprenant un ou plusieurs parmi l'oméga-3, l'oméga-6 et l'oméga-9 ou un dérivé de ceux-ci et l'antioxydant sont administrés de manière simultanée.

26. Combinaison médicamenteuse comprenant essentiellement :
du budesonide ou un promédicament ou un dérivé de celui-ci,
un acide gras polyinsaturé comprenant un ou plusieurs parmi l'oméga-3, l'oméga-6 et l'oméga-9 ou un dérivé de ceux-ci, et
un antioxydant pharmacologiquement actif.

27. Combinaison médicamenteuse suivant la revendication 26, comprenant en outre un vecteur pharmaceutiquement acceptable comprenant un ou plusieurs parmi d'autres huiles essentielles, des huiles non essentielles, des diluants, des modificateurs de la viscosité, des stabilisants, des matrices pouvant être érodées, des matrices pouvant être avalées, et des agents améliorant la pénétration.

28. Combinaison médicamenteuse suivant la revendication 26 ou 27, dans lequel l'antioxydant pharmacologiquement actif comprend du tocophérol.

29. Combinaison médicamenteuse suivant la revendication 26 ou 27, dans lequel l'antioxydant pharmacologiquement actif comprend un mélange d'isomères alpha et gamma du tocophérol.

30. Combinaison médicamenteuse suivant l'une quelconque des revendications 26-29, dans lequel l'acide gras polyinsaturé est une huile purifiée présentant au moins 50% de sa teneur en lipides comprenant l'acide gras polyinsaturé oméga-3 et/ou l'oméga-6 et/ou l'oméga-9.

31. Combinaison médicamenteuse suivant l'une quelconque des revendications 26-30, dans lequel l'acide gras polyinsaturé oméga-3, oméga-6, oméga-9 ou un dérivé de ceux-ci est sous une forme biodisponible.

32. Combinaison médicamenteuse suivant la revendication 31, dans lequel la forme biodisponible est un tri-, un di- ou un monoglycéride, un méthyl/éthylester, ou un acide gras libre.

33. Combinaison médicamenteuse suivant l'une quelconque des revendications 26-32, dans lequel le budesonide, ou un promédicament ou un dérivé de celui-ci, est entièrement en solution.

34. Combinaison médicamenteuse suivant l'une quelconque des revendications 26-32, dans lequel le budesonide, ou un promédicament ou un dérivé de celui-ci, est entièrement en suspension.

35. Combinaison médicamenteuse suivant l'une quelconque des revendications 26-32, dans lequel le budesonide, ou un promédicament ou un dérivé de celui-ci, est partiellement en suspension.

36. Combinaison médicamenteuse suivant l'une quelconque des revendications 26-35, dans lequel la composition procure de 0,1 à 30 grammes d'acide gras polyinsaturé oméga-3 et/ou oméga-6 et/ou oméga-9, ou d'un dérivé de ceux-ci.

37. Combinaison médicamenteuse suivant l'une quelconque des revendications 26-36, dans lequel le kit de parties peut être administré par voie orale, entérale ou topique.

38. Combinaison médicamenteuse suivant l'une quelconque des revendications 26-37, dans lequel la combinaison médicamenteuse est adaptée pour une administration simultanée.
